(19) **Europäisches Patentamt European Patent Office Office européen des brevets**

(11) **EP 2 548 547 B1**

(12) **EUROPEAN PATENT SPECIFICATION**

(45) Date of publication and mention of the grant of the patent:
**23.11.2016 Bulletin 2016/47**

(51) Int Cl.:
*A61K 8/18* (2006.01)    *A61K 8/73* (2006.01)
*A61Q 11/00* (2006.01)

(21) Application number: **12185405.3**

(22) Date of filing: **22.03.2005**

(54) **Composition for toothbrushing**

Zahnputzzusammensetzung

Composition pour dentifrice

(84) Designated Contracting States:
**DE FR GB**

(30) Priority: **19.03.2004 JP 2004079679**
          **21.05.2004 JP 2004152143**
          **03.09.2004 JP 2004257541**
          **03.09.2004 JP 2004257542**

(43) Date of publication of application:
**23.01.2013 Bulletin 2013/04**

(62) Document number(s) of the earlier application(s) in accordance with Art. 76 EPC:
**05727074.6 / 1 726 288**

(73) Proprietor: **KAO CORPORATION**
**Tokyo 103-8210 (JP)**

(72) Inventors:
• **Yoshida, Hidenori**
  **Tokyo, 131-8501 (JP)**
• **Murakami, Yoshinori**
  **Tokyo, 131-8501 (JP)**
• **Oshino, Kazushi**
  **Tokyo, 131-8501 (JP)**

(74) Representative: **Hoffmann Eitle**
**Patent- und Rechtsanwälte PartmbB**
**Arabellastraße 30**
**81925 München (DE)**

(56) References cited:
**EP-A1- 0 480 172    WO-A1-95/34275**
**GB-A- 2 252 042    JP-A- 1 299 211**
**JP-A- 9 012 436    JP-A- 55 098 111**

Remarks:
The file contains technical information submitted after the application was filed and not included in this specification

**Description**

[Technical field]

The present invention relates to a dentifrice composition excellent in plaque and stain removing effects and also feeling upon use.

[Background Art]

As a dentifrice containing cellulose therein, a dentifrice composition that contains a cellulose powder having an average particle size of from 0.005 to 1 mm is disclosed as a substitute for an abrasive (Patent Document 1). Such a cellulose powder, however, cannot be said to possess sufficient plaque removing power, so that use of a granulated cellulose having an average particle size of from 50 to 1000 μm was proposed in lieu of such a cellulose powder (Patent Document 2). Nevertheless, such a granulated cellulose is still not sufficient in color-removing effects, and thus there has been a demand for use of non-granulated cellulose from the viewpoint of its production cost.

From the viewpoints of detergency and feeling upon use, there has also been a demand for a dentifrice composition enabling an adequate amount of foam to last, thereby making it easier to brush the teeth for a long period of time. With regard to foaming, a high foaming property is usually preferred. The foaming property or foaming rate of various surfactants has been investigated conventionally. In order to heighten the foaming property, there is a proposal indicating that the foaming amount should be increased, or a proposal indicating that foams with a high shape retention should be maintained for long hours (Patent Document 3). However, no report has yet been made on the relationship between the foam quality and foaming amount of cellulose powder and a dentifrice composition containing it.

[Patent Document 1] JP-A-55-98111
[Patent Document 2] JP-A-09-40537
[Patent Document 3] JP-11-209255

EP 0 480 172 A1 relate to a preparation for the care of the teeth consisting of the customary known ingredients of a preparation for the care of the teeth and containing as abrasive component cellulose powder in place of customary known polishing particles.

WO 95/34275 A1 relates to an oral hygiene composition, especially a tooth-whitening composition, comprising a silica abrasive and a particulate cellulose cleaning/polishing agent (particle size less than 50 μm). GB 2 252 042 A relate to a composition for use in the oral cavity comprising granules composed of a water-insoluble powder and a water-insoluble organic binder; and which granules have an apparent density of from 0.1 to 1.5 and collapse at a deformation rate of from 0.1 to 20% at a load of from 0.1 to 50 g per granule.

JP 1 299211 A relates to a dentifrice which is prepared by compounding a granule obtained by granulating a water-insoluble powdery material having particle diameter of about 0.1-20 μm and used as a conventional tooth polishing material (e.g., calcium hydrogen phosphate, insoluble sodium metasilicate, silica or zeolite) with a water-insoluble inorganic binder (preferably magnesium metasilicate aluminate or colloidal silica) to obtain a granule having particle size passing through a 30 mesh sieve but incapable of passing through a 200 mesh sieve and collapsing under a load of 0.1-10g per one granule. JP 9 12436 A relates to a dentifrice containing 1-30 wt.% of wet method silica particles which have particle sizes passing through a 30 mesh sieve but not passing through a 200 mesh sieve, a collapse strength of 30-800gf/mm$^2$/particle, and a polishing power expressed by a RDA value of ≤150.

[Disclosure of the Invention]

In the present invention, there is thus provided a dentifrice composition containing the following components (A), (B) and (D):

(A) cellulose powder
(B) a surfactant, and
(D) granules having a particle size permitting passage through a 30-mesh (JIS standard) sieve but not permitting passage of a 200-mesh (JIS standard) sieve, wherein the powder cellulose (A) is a non-granulated cellulose powder having an average particle size of 10 to 50 μm.

[Brief Description of the Drawings]

[FIG. 1] FIG. 1 is a schematic view illustrating an interdental model used for a stain removal test and an interdental space to be evaluated.

[FIG. 2] FIG. 2 is a graph showing the results of the stain removal test.

[FIG. 3] FIG. 3 is a schematic view of a brushing machine.

[Mode for Carrying out the Invention]

The present inventors have carried out an extensive investigation to obtain a dentifrice composition excellent in stain removing effect and also in feeling upon use.

As the cellulose powder (A) to be used in the present invention, one or more kinds obtained by chemically treating celluloses such as pulp powder, insoluble powder cellulose, powdered $\alpha$-cellulose and pulp to insolubilize them and then grinding them can be used. From the standing points of stain removal effect, the cellulose powder has preferably an average polymerization degree of 350 or greater, more preferably from about 350 to 2250, still more preferably from about 440 to 2550. From the viewpoints of a fine touch feel and a foam retention, the cellulose powder is preferably a non-granulated cellulose powder having an average particle size of from 10 to 50 $\mu$m, more preferably from 15 to 50 $\mu$m.

The content of the cellulose powder in the dentifrice composition is preferably from 0.2 to 3 weight% in the whole composition. From the viewpoints of improving a stain removing effect and preparing viscous foams, the content is preferably 0.2 weight% or greater, more preferably 0.4 weight% or greater. From the viewpoint of the feeling of the dentifrice composition upon use, the content is preferably 3 weight% or greater, more preferably 2 weight% or greater, especially preferably 1 weight% or greater.

The dentifrice composition of the present invention may be prepared by adding the cellulose powder in the powder form or in the form of a dispersion obtained by dispersing the cellulose powder in a liquid such as water, a lower alcohol or polyol. Examples of the lower alcohol include ethanol and isopropanol, while those of the polyol include glycerin, polyethylene glycol and propylene glycol. When it is prepared, use of a dispersion having the cellulose powder dispersed in water, a lower alcohol or a polyol is preferred.

The cellulose powder itself has almost no abrasive powder, but small amount of addition considerably enhances the action of an abrasive. This is presumed to occur because owing to the existence of the cellulose powder between bristles and abrasive grains of a teeth brush, the cellulose powder contributes to interaction (friction) between abrasive grains and tooth surface. The cellulose powder is presumed to serve also as a buffer material and exhibits selective detergency to remove stain without much wear of dentin so that it is advantageous. Particularly when an abrasive with low RDA is used, a large amount of an ordinarily used abrasive must be added. Addition of the cellulose powder however can reduce the amount of an abrasive.

The dentifrice composition of the present invention contains a surfactant (B) such as an anionic surfactant, an nonionic surfactant or an amphoteric surfactant. Of these, an anionic surfactant is preferred.

No particular limitation is imposed on the anionic surfactant insofar as it is an anionic surfactant ordinarily used for dentifrice compositions. Examples include acylaminoacid salts such as sodium acylglutamate and acylsalcosin sodium, alkylphosphates such as sodium alkylphosphate, alkyl sulfate ester salts, higher fatty acid sulfonated monoglyceride salts, fatty ace ester salts of isothionic acid, N-methyl(long chain acyl)taurin sodium salts and polyoxyethylene monoalkyl phosphates.

Anionic surfactants having, in the hydrophobic group thereof, an acyl or alkyl group having from 6 to 18 carbon atoms, especially preferably from 10 to 14 carbon atoms are preferred, of which sodium salts are preferred. Alkyl sulfate esters are especially preferred because they are available at a low cost. The anionic surfactant is preferably added in an amount of from 0.1 to 5 weight%, especially preferably from 0.1 to 2 weight% in the dentifrice composition of the present invention.

No particular limitation is imposed on the nonionic surfactant insofar as it is a nonionic surfactant ordinarily used for dentifrice compositions. Examples include polyoxyalkylene addition surfactants, amine-oxide surfactants, mono- or diethanolamide surfactants, sucrose fatty acid esters, glycerin fatty acid esters, polyglycerin fatty acid esters, sorbitan fatty acid esters, polyoxyethylene sorbitan fatty acid esters, and polyoxyethylene hydrogenated castor oils. Of these, sucrose fatty acid esters, glycerin fatty acid esters, polyglycerin fatty acid esters, sorbitan fatty acid esters, polyoxyethylene sorbitan fatty acid esters, polyoxyethylene hydrogenated castor oils are preferred. In the present invention, one or more of them may be used. Nonionic surfactants having an HLB number (Griffin) of 16 or greater, especially from 18 to 20 are preferred.

(Equation 1)

$$\text{HLB number} = 20(1-S/A)$$

(wherein, S: saponification number, A: acid number of a fatty acid employed.

The fatty acid moiety of these nonionic surfactants has preferably from 6 to 18 carbon atoms. Its content is preferably from 0.1 to 30 wt.%, more preferably from 0.2 to 10 wt.%, still more preferably fro 1.2 to 3.0 wt.%, still more preferably from 1.3 to 2.5 wt.%, especially preferably from 1.3 to 2.0 wt.%. The dentifrice composition preferably does not contain a nonionic surfactant having an HLB number of 15 or less in order to obtain viscous foams. When the nonionic surfactant having an HLB number of 15 or less is added, its amount is preferably 0.5 parts by mass, more preferably 0.3 parts by mass or less, each relative to 1 part by mass of the anionic surfactant.

No particular limitation is imposed on the amphoteric surfactant insofar as it is an amphoteric surfactant ordinarily employed for dentifrice compositions. Examples include alkyl betaine, alkyl amidobetaine, imidazoline and glycine. Its content is preferably from 0.01 to 10 weight%, more preferably from 0.05 to 5 weight% in the whole dentifrice composition.

The dentifrice composition of the present invention contains (D) granules. The granules (D) to be used in the present invention have a particle size (from 75 to 500 $\mu$m) which passes through a 30-mesh sieve (JIS standards) but does not pass through a 200-mesh sieve (JIS standards). Examples of such granules include granules available by binding water insoluble powder materials with a water insoluble inorganic binder as described in JP-B-06-021053, granules available by coagulating only fine particles of calcium carbonate as described in Japanese Patent No. 3170250, granules available by binding water insoluble powder materials with a water insoluble organic binder as described in JP-04-243815, and wet-method silica granules having a specific surface area, as measured by the BET method, of from 150 to 450 $m^2$/g as described in JP-09-12436. Those having a disintegration strength when 0.1 to 30 g of a load is applied per granule are preferred from the viewpoint of feeling upon use. The content of the granules is preferably from 0.5 to 30 weight%, more preferably from 1 to 30 weight%, still more preferably from 1 to 25 weight%, still more preferably from 2 to 20 weight% in the whole dentifrice composition.

The dentifrice composition of the present invention may contain a polyphosphate further. Examples of the polyphosphate include linear polyphosphates such as sodium pyrophosphate, potassium pyrophosphate, sodium tripolyphosphate, potassium tripolyphosphate, sodium tetrapolyphosphate, potassium tetrapolyphosphate and sodium metaphosphate and cyclic polyphosphates such as sodium trimetaphosphate, potassium trimetaphosphate, sodium tetrametaphosphate, potassium tetrametaphosphate, sodium hexametaphosphate and potassium hexametaphosphate. These polyphosphates may be used either singly or as a mixture of two or ore of them. Of these, linear polyphosphates are preferred, with those having a polymerization of from 2 to 4 being especially preferred. The content of the polyphosphate is preferably from 0.05 to 10 weight%, more preferably from 0.1 to 8 weight%, even more preferably from 0.1 to 5 weight%.

The dentifrice composition of the present invention may further contain polyethylene glycol. The polyethylene glycol to be incorporated in the dentifrice composition of the present invention has preferably an average molecular weight of from 200 to 1000 and its content is preferably from 1 to 10 weight%, more preferably from 2 to 8 weight%, even more preferably from 3 to 7 weight% in the whole composition.

The dentifrice composition according to the present invention comprises (A) cellulose powder, (B) a surfactant and (D) granules having a specific particle size. The dentifrice composition excellent in the removal of stain, providing foams with a fine touch feel during use and capable of retaining foams for a long time can be provided by containing such components. The components (A), (B) and (D) to be added to the dentifrice composition of the present invention are described above.

The dentifrice composition of the present invention preferably further contains a binder. It is preferred to add one or more binders selected from the group consisting of sodium alginate, sodium carboxymethyl cellulose, carrageenan, xanthan gum, sodium polyacrylate, hydroxyethyl cellulose, hydroxypropyl cellulose, pectin, tragacanth gum, arabic gum, guar gum, karaya gum, locust bean gum, gellan gum, tamarind gum, Psyllium seed gum, polyvinyl alcohol, sodium chondroitin sulfate, and methoxyethylene maleic anhydride copolymer. Of these, sodium alginate, carboxymethyl cellulose, carrageenan, and xanthan gum are especially preferred. The content of the binder is preferably from about 0.1 to 3 weight%, more preferably from 0.2 to 2 weight%, still more preferably from 0.2 to 1.5, even more preferably from 0.5 to 1.5 weight% in the whole dentifrice composition.

The dentifrice composition of the present invention can contain, in addition to the above-described components, for example, a foaming agent, a foaming assistant, an abrasive, a humectant, a sweetener, a preservative, an enzyme, a pH regulator, a bactericide, a pharmaceutically effective component, a pigment, a colorant and flavor as needed.

Preferred examples of the humectant include glycerin, sorbitol, propylene glycol, polyethylene glycol, xylitol, maltitol, lactitol and trehalose. The content of the humectant, if any, is preferably fro 1 to 10 weight%, more preferably from 2 to 8 weight%, even more preferably from 3 to 7 weight% in the whole dentifrice composition.

Examples of the sweetener include saccharin sodium, aspartame, thaumatin, acesulfame potassium, stevioside, stevia extract, paramethoxy cinnamic aldehyde, neohesperidin dihydrochalcon and perillartine.

Examples of the pH regulator include phosphoric acid and salts thereof (such as sodium phosphate and sodium hydrogen phosphate), citric acid and salts thereof (such as sodium citrate), malic acid and salts thereof, gluconic acid and salts thereof, maleic acid and salts thereof, aspartic acid and salts thereof, succinic acid and salts thereof, glucuronic acid and salts thereof, fumaric acid and salts thereof, glutamic acid and salts thereof, adipic acid and salts thereof, hydrochloric acid, sodium hydroxide, potassium hydroxide and sodium silicate. Although there is no particular limitation imposed on the content of the pH regulator insofar as the composition has a desired pH, it is usually added in an amount of from about from 0.01 to 5 weight%, preferably from about 0.1 to 3 weight% based on the whole composition. Although no particular limitation is imposed on the pH of the dentifrice composition of the present invention insofar as it can exhibit the advantages of the present invention, it is usually from about 4 to 10.

Examples of the flavor include 1-menthol, carvone, anethole, eugenol, limonene, peppermint oil, spearmint oil, ocimene, n-amyl alcohol, citronellol, $\alpha$-terpineol, methyl salicylate, methyl acetate, acetate, cineol, linalool, ethyl linalool, vanillin, thymol, lemon oil, orange oil, sage oil, rosemary oil, cinnamon oil, pimento oil, perilla oil, clove oil and eucalyptus oil.

Examples of the other various medicinally effective ingredients include water-soluble phosphoric acid compounds such as potassium salt or sodium salt of orthophosphoric acid, allantoin chlorohydroxyaluminum, hinokitiol, lysozyme chloride, glycyrrhizinic acid and salts thereof, sodium chloride, tranexamic acid, epsilon-aminocaproic acid, d1-tocopherol acetate, azulene, glycyrrhetinic acid, copper compounds such as sodium copper chlorophyllin and copper gluconate, aluminum lactate, strontium chloride, potassium nitrate, berberine, hydroxamic acid and derivatives thereof, sodium tripolyphosphate, zeolite, dextranase, mutanase, amylase, methoxyethylene, maleic anhydride copolymer, polyvinylpyrrolidone, epidihydrocholesterin, dihydrocholesterol, zinc citrate, extracts of Japanese angelica roots, phellodendron barks, clove, rosemary, scutellaria roots, safflower, and the like, benzethonium chloride, trichlorocarbanilide, $\alpha$-bisabolol, chlorhexidine salts, triclosan, cetylpyridinium chloride, benzethonium chloride, and trichlorocarbanilide.

The water content can be determined as needed depending on the form of the composition, but it is usually from about 0 to 60 weight%, preferably from about 10 to 50 weight% based on the whole dentifrice composition.

[Examples]

The present invention will hereinafter be described in full detail by Examples and Comparative Examples. It should however be borne in mind that the present invention is not limited to or by these Examples. All designations of "%" mean weight% unless otherwise specifically indicated.

Examples 1 to 5 and Comparative Examples 1 and 2

Toothpastes were prepared in accordance with the formulations as described in Table 1.

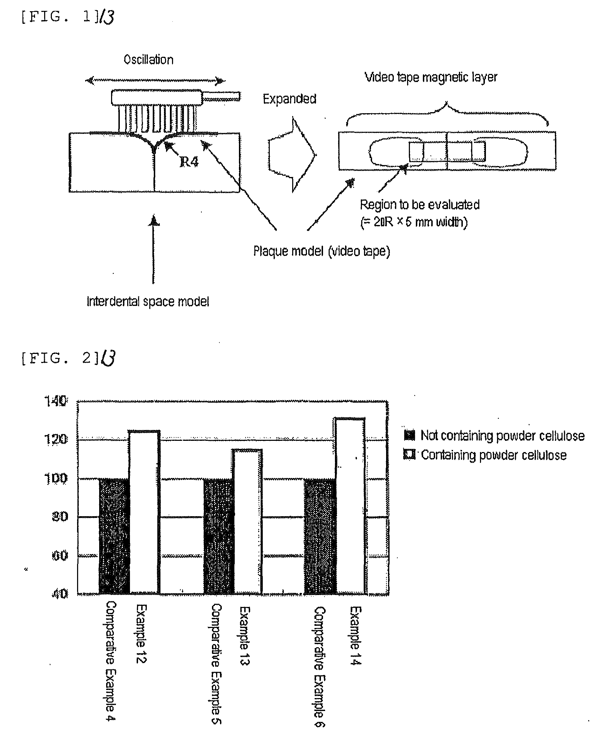

The dentifrice compositions thus obtained was tested to know their stain removing effect at the interdental space. Described specifically, as illustrated in FIG. 1, an interdental space model was formed using aluminum blocks while facing their R4 curved surfaces each other. A video tape magnetic layer was attached as a colored model to the surface of the resulting interdental space model. By using a brushing machine as illustrated in FIG. 2, the interdental model thus formed was cleaned with each of the toothpastes obtained in Examples 1 to 5 and those obtained in Comparative Examples 1 and 2. A toothbrush ("Check Standard", product of Kao) was used for brushing under the following conditions: load of 300 g, rate of 120 r/min, amplitude of 30 mm and 120 brushing times. A region of $2\pi$R4 $\times$ 5 mm width was evaluated as an interdental space. After cleaning by brushing, the video tape magnetic layer was expanded, photographed by a digital camera and subjected to image analysis. An area (mm$^2$) of a portion which had become white because the magnetic layer was peeled from the evaluation region was calculated by the image analysis and the whitening and stain removal ratio at the interdental space was evaluated. The evaluation results are shown in Table 1. With the whitening and stain removal effects at the interdental space in Comparative Example 1 as a reference (100%), a removal ratio relative thereto was indicated as evaluation results.

| [Table 1] | | | | | | | (weight%) |
|---|---|---|---|---|---|---|---|
| (weight%) | Examples | | | | | Comparative Examples | |
| | 1 | 2 | 3 | 4 | 5 | 1 | 2 |
| Abrasive silica *1 | 20.00 | 20.00 | 20.00 | 20.00 | 19.00 | 20.00 | 20.00 |
| Powder cellulose (average particle size: 20$\mu$m) | 0.50 | | | | 2.00 | | |
| Powder cellulose (average particle size: 30$\mu$m) | | 0.50 | | | | | |

EP 2 548 547 B1

(continued)

| (weight%) | Examples | | | | | Comparative Examples | |
|---|---|---|---|---|---|---|---|
| | 1 | 2 | 3 | 4 | 5 | 1 | 2 |
| Powder cellulose (average particle size: 40μm) | | | 0.50 | | | | |
| Powder cellulose (average particle size: 50μm) | | | | 0.50 | | | |
| Crystalline cellulose (average particles size: 10 μm or less) | | | | | | | 0.50 |
| Polyethylene glycol(PEG600) | 5.00 | 5.00 | 5.00 | 5.00 | 5.00 | 5.00 | 5.00 |
| Sodium lauryl sulfate | 1.50 | 1.50 | 1.50 | 1.50 | 1.50 | 1.50 | 1.50 |
| Viscous silica | 3.00 | 3.00 | 3.00 | 3.00 | 3.00 | 3.00 | 3.00 |
| Xanthan gum | 0.50 | 0.50 | 0.50 | 0.50 | 0.50 | 0.50 | 0.50 |
| Carrageenan | 0.50 | 0.50 | 0.50 | 0.50 | 0.50 | 0.50 | 0.50 |
| Concentrated glycerin | 20.00 | 20.00 | 20.00 | 20.00 | 20.00 | 20.00 | 20.00 |
| Sorbitol | 30.00 | 30.00 | 30.00 | 30.00 | 30.00 | 30.00 | 30.00 |
| Saccharin sodium | 0.15 | 0.15 | 0.15 | 0.15 | 0.15 | 0.15 | 0.15 |
| Sodium fluoride | 0.20 | 0.20 | 0.20 | 0.20 | 0.20 | 0.20 | 0.20 |
| DL-malic acid | 2.00 | 2.00 | 2.00 | 2.00 | 2.00 | 2.00 | 2.00 |
| Potassium hydroxide solution (48wt.%) | 2.50 | 2.50 | 2.50 | 2.50 | 2.50 | 2.50 | 2.50 |
| Sodium hydroxide solution (48 wt.%) | 0.50 | 0.50 | 0.50 | 0.50 | 0.50 | 0.50 | 0.50 |
| Titanium oxide | 0.30 | 0.30 | 0.30 | 0.30 | 0.30 | 0.30 | 0.30 |
| Flavor | 1.00 | 1.00 | 1.00 | 1.00 | 1.00 | 1.00 | 1.00 |
| Purified water | Balance | Balance | Balance | Balance | Balance | Balance | Balance |
| | 100 | 100 | 100 | 100 | 100 | 100 | 100 |
| Stain removing effect at the interdental space (%) | 130 | 128 | 131 | 135 | 129 | 100 | 113 |

[Table 1] (weight%)

*1:RDA(110~150)

As is apparent from Table 1, the compositions of Examples 1 to 5 containing powder cellulose showed a superior stain removing effect at the interdental space to the composition of Comparative Example 1 not containing powder cellulose. The composition of Comparative Example 2 containing crystalline cellulose had improved the stain removing effect at the interdental space, but the effect was inferior to that of the composition containing powder cellulose.

Examples 6 to 11 and Comparative Example 3

Oral compositions were prepared in accordance with the formulation shown in Table 2.

| [Table 2] | | | | | | | (weight%) |
|---|---|---|---|---|---|---|---|
| (weight%) | Examples | | | | | | Comp. Ex. |
| | 6 | 7 | 8 | 9 | 10 | 11 | 3 |
| Aluminum hydroxide | | 20.00 | | | | | |
| Alumina | | 5.00 | | | | | |
| Potassium hydrogen phosphate dihyrate | | | | 30.00 | | 20.00 | |
| Anhydrous potassium hydrogen phosphate | | | | | | 10.00 | |
| Abrasive silica (RDA:150~200) | | | | | 3.00 | | |
| Abrasive silica (RDA:110~150) | 16.00 | | | | 13.00 | | 3.00 |
| Abrasive silica (RDA:値20~110) | | | 25.00 | | | | |
| Powder cellulose (average particle size: 20μm) | 0.50 | 0.50 | 0.50 | | 0.30 | | |
| Powder cellulose (average particle size: 50μm) | | | | 0.20 | | 1.00 | |
| Powder cellulose | | | | | | | 20.0 |
| Polyethylene glycol(PEG600) | 5.00 | | 5.00 | | | | 5.00 |
| Sodium lauryl sulfate | 1.50 | 1.50 | | 1.60 | 1.00 | 1.60 | 1.50 |
| Sodium N-lauroyl-L-glutamate | | | | | | 0.30 | |
| POE (20) sorbitan monostearate | | | 0.30 | | | | |
| POE(60) hydrogenated castor oil | | | | | | 1.00 | |
| Glycerin fatty acid ester | | | 1.50 | | | | |
| Thickening silica | 4.00 | 4.00 | 4.00 | 1.00 | 5.00 | | |
| Sodium polyacrylate | | | | | | 0.50 | |
| Xanthan gum | 0.20 | 0.30 | 0.30 | | 0.30 | | 0.50 |
| Carboxymethylcellulose sodium | | 0.50 | | 0.30 | | 0.30 | |
| Carrageenan | 0.60 | | | 0.20 | 0.30 | | 0.50 |
| Sodium alginate | | | 0.50 | | | | |
| Propylene glycol | | | | | 4.00 | | |
| Concentrated glycerin | 15.00 | 30.00 | 15.00 | 15.00 | 20.00 | 25.00 | 25.00 |
| Sorbitol | 30.00 | 15.00 | 20.00 | 30.00 | 30.00 | 25.00 | 23.00 |
| Saccharin sodium | 0.15 | 1.30 | 0.15 | 0.12 | 0.15 | 0.12 | 0.12 |
| Sodium fluoride | 0.20 | | 0.20 | | 0.20 | | 0.20 |

(continued)

| [Table 2] | | | | | | | (weight%) |
|---|---|---|---|---|---|---|---|
| (weight%) | Examples | | | | | | Comp. Ex. |
| | 6 | 7 | 8 | 9 | 10 | 11 | 3 |
| Sodium monofluorophosphate | | 0.70 | | 0.70 | | 0.70 | |
| dl-a-tocopherol acetate | | | | | 0.30 | | |
| DL-malic acid | 2.00 | 2.00 | 2.20 | | | | 2.00 |
| Potassium hydroxide solution (48 weight%) | | | 2.50 | | | | |
| Sodium hydroxide solution (48 weight%) | 2.50 | 2.50 | | | | | 2.50 |
| L-arginine | | | 1.20 | | | | |
| Triclosan | | 0.10 | | | | 0.10 | |
| Benzethonium chloride | | | | 0.01 | | | |
| Titanium oxide | 0.30 | | 0.30 | 0.30 | 0.30 | | 0.30 |
| Zinc chloride | | | | 0.50 | | | |
| Flavor | 1.00 | 1.00 | 1.00 | 1.00 | 1.00 | 1.00 | 1.00 |
| Purified water | Balance | Balance | Balance | Balance | Balance | Balance | Balance |
| | 100 | 100 | 100 | 100 | 100 | 100 | 100 |

The dentifrice compositions of the present invention obtained in Examples 6 to 11 exhibited, similar to those obtained in Examples 1 to 5, an excellent stain removing effect at the interdental space, but the composition obtained in Comparative Example 3 exhibited a stain removing effect as low as 0.3%.

Examples 12 to 14 and Comparative Examples 3 to 5

Toothpastes were prepared in accordance with the formulations as described in Table 3.

| [Table 3] | | | | | | (weight%) |
|---|---|---|---|---|---|---|
| | Ex. 12 | Comp. Ex. 4 | Ex. 13 | Comp. Ex. 5 | Ex. 14 | Comp. Ex. 6 |
| Abrasive silica (RDA:110-150) | 16.00 | 16.00 | 16.00 | 16.00 | 16.00 | 16.00 |
| Powder cellulose (average particle size: 50μm) | 0.50 | | 0.50 | | 0.50 | |
| Silica granules (average particle size: 200μm) | 3.00 | 3.00 | | | | |
| Sodium tripolyphosphate (STPP) | | | 0.10 | 0.10 | | |
| Polyethylene glycol (PEG600) | | | | | 5.00 | 5.00 |
| Sodium lauryl sulfate | 1.50 | 1.50 | 1.50 | 1.50 | 1.50 | 1.50 |
| Thickening silica | 4.00 | 4.00 | 4.00 | 4.00 | 4.00 | 4.00 |
| Xanthan gum | 0.20 | 0.20 | 0.20 | 0.20 | 0.20 | 0.20 |
| Carrageenan | 0.60 | 0.60 | 0.60 | 0.60 | 0.60 | 0.60 |
| Propylene glycol | 5.00 | 5.00 | 5.00 | 5.00 | | |

(continued)

| [Table 3] | | | | | | (weight%) |
|---|---|---|---|---|---|---|
| | Ex. 12 | Comp. Ex. 4 | Ex. 13 | Comp. Ex. 5 | Ex. 14 | Comp. Ex. 6 |
| Concentrated glycerin | 20.00 | 20.00 | 20.00 | 20.00 | 20.00 | 20.00 |
| Sorbitol | 30.00 | 30.00 | 30.00 | 30.00 | 30.00 | 30.00 |
| Saccharin sodium | 0.15 | 0.15 | 0.15 | 0.15 | 0.15 | 0.15 |
| Sodium fluoride | 0.20 | 0.20 | 0.20 | 0.20 | 0.20 | 0.20 |
| DL-Malic acid | 2.00 | 2.00 | 2.00 | 2.00 | 2.00 | 2.00 |
| Sodium hydroxide solution (48 weight%) | 2.50 | 2.50 | 2.50 | 2.50 | 2.50 | 2.50 |
| Titanium oxide | 0.30 | 0.30 | 0.30 | 0.30 | 0.30 | 0.30 |
| Flavor | 1.00 | 1.00 | 1.00 | 1.00 | 1.00 | 1.00 |
| Purified water | Balance | Balance | Balance | Balance | Balance | Balance |
| | 100 | 100 | 100 | 100 | 100 | 100 |

In a similar manner to Example 1, the toothpastes thus obtained were tested for the stain removing effect at the interdental space. The evaluation results are shown in FIG. 2. With the stain removing effect at the interdental space in Comparative Examples 3 to 5 corresponding to Examples 12 to 14, respectively as a reference (100%), the evaluation results of them were indicated by a relative removing ratio.

As is apparent from FIG. 2, the toothpastes of Examples 12 to 14 containing powder cellulose were superior in stain removing effect at the interdental space to those of Comparative Examples 3 to 5 not containing powder cellulose.

Example 15

Toothpastes were prepared in accordance with the formulations as shown in Table 4.

[Table 4]

| | Example 15 | Comp. Ex. 7 | Comp. Ex.8 |
|---|---|---|---|
| Calcium carbonate | | 50.00 | 5.00 |
| Silicic anhydride | 18.50 | | 7.00 |
| Powder cellulose (average particle size: about 50 $\mu$m) | 0.50 | | |
| Polyethylene glycol (PEG600) | 5.00 | | 5.00 |
| Silicic anhydride granules (average particle size: 200 $\mu$m) | 2.50 | | 14.00 |
| Sodium lauryl sulfate | 1.50 | 1.50 | 1.50 |
| Xanthan gum | 0.40 | | 0.20 |
| Carrageenan | 0.60 | 0.50 | |
| Carboxylmethylcellulose sodium | | 1.00 | 1.50 |
| Concentrated glycerin | 20.00 | 4.00 | |
| Sorbitol (sorbitol solution) | 30.00 | 15.00 | 36.00 |
| Saccharin sodium | 0.15 | 0.15 | 0.15 |
| Sodium fluoride | 0.20 | | |
| Sodium monofluorophosphate | | 0.70 | 0.70 |
| DL-Malic acid | 2.00 | | |
| pH Regulator | Amount to adjust pH to 6.0 | | |

(continued)

|  | Example 15 | Comp. Ex. 7 | Comp. Ex.8 |
|---|---|---|---|
| Flavor | 1.40 | 0.85 | 1.00 |
| Purified water | Balance | Balance | Balance |
| Total | 100 | 100 | 100 |

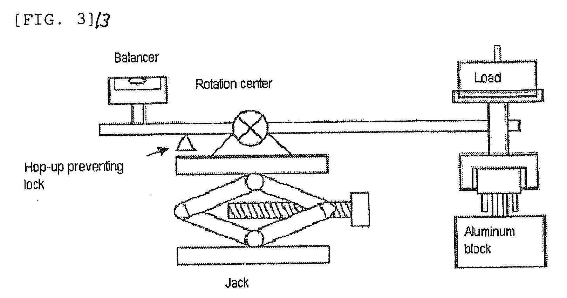

The dentifrice compositions thus obtained were tested for their foaming property. A surface to be brushed was formed by arranging, on an acrylic plate (30 × 80 × 3 mm), columnar-shaped acrylic rods having $\varphi$ 3 vertically relative to the pedestal running direction. An acrylic vessel (80 × 175 × 35 mm) was attached to fix the surface. The toothpastes of Example 15 and toothpastes of Comparative Examples 6 and 7 were each diluted to 5 times the original weight and a foaming test of the diluted toothpaste was performed by moving a brushing machine (FIG. 3, having a horizontally movable pedestal) on the surface to be brushed.

Cleaning by brushing was conducted by using a toothbrush ("Kesakigakyu", trade name; product of Kao) under a load of 500 g, rate of 120 r/min, amplitude of 50 mm and cleaning of 500 times. The liquid remaining after cleaning was transferred to a measuring cylinder and the foam amount was measured. The amount (mL) of foams present in the upper part of the measuring cylinder is shown in Table 5 as the evaluation results of the foaming property.

[Table 5]

| Foam volume (mL) | Time after tooth brushing (minute) | |
|---|---|---|
| | 1 | 15 |
| Example 15 | 61 | 60 |
| Comp. Ex. 7 | 56 | 52.5 |
| Comp. Ex. 8 | 54 | 52 |

A panel of five experts was asked to use each toothpaste and organoleptically evaluate the foam quality at the time of using from the viewpoints of texture of foam, wateriness of foam, viscosity of foam and foaming ease based on the below-described criteria.

(1) Texture of foam

Fine: 2
Rather fine 1:
Difficult to evaluate: 0
Rather coarse: -1
Coarse: 1

(2) Elasticity of foam

Elastic: 2
Rather elastic: 1
Difficult to evaluate: 0
Slightly watery: -1
Watery: -2

(3) Viscosity of foam

Viscous: 2
Rather viscous: 1
Difficult to evaluate: 0
Slightly viscous: -1
Not viscous: -2

(4) Foaming property at the time of use

Prompt: 2
Rather prompt: 1
Difficult to evaluate: 0
Rather slow: -1
Slow: -2

The total evaluation scores of five experts are shown in Table 6.

[Table 6]

|  | Texture of foam | Wateriness of foam | Viscosity of foam | Foaming property |
|---|---|---|---|---|
| Example 15 | 5 | 6 | 3 | 3 |
| Comparative Example 7 | -1 | 4 | 1 | 2 |
| Comparative Example 8 | 0 | -2 | -2 | 1 |

It has been understood that the toothpaste of the present invention (Example 15) had a good foaming property and good foam quality with fine texture. The toothpaste containing neither powder cellulose nor cellulose granules (Comparative Example 7) and the toothpaste not containing powder cellulose (Comparative Example 8) were poor in both foaming property and foam quality.

**Claims**

1. A dentifrice composition comprising the following components (A), (B) and (D):

    (A) powder cellulose;
    (B) a surfactant, and
    (D) granules having a particle size permitting passage of a 30-mesh (JIS standard) sieve but not permitting passage of a 200-mesh sieve (JIS standard),
    wherein the powder cellulose (A) is a non-granulated cellulose powder having an average particle size of 10 to 50 μm.

2. The dentifrice composition according to claim 1, wherein the content of the powder cellulose is from 0.2 to 3 weight%.

3. The dentifrice composition according to Claim 1 or Claim 2, wherein the content of the powder cellulose is from 0.4 to 2 weight%.

4. The dentifrice composition according to any one of Claims 1 to 3, wherein the powder cellulose has an average polymerization degree of from 350 to 2250.

5. The dentifrice composition according to any one of Claims 1 to 4, further comprising a binder.

6. The dentifrice composition according to Claim 5, wherein the binder comprises at least two binders selected from the group consisting of sodium alginate, sodium carboxymethyl cellulose, carrageenan, xanthan gum, sodium 1 polyacrylate, hydroxyethyl cellulose, hydroxypropyl cellulose, pectin, tragacanth gum, arabic gum, guar gum, karaya gum, locust bean gum, gellan gum, tamarind gum, Psyllium seed gum, polyvinyl alcohol, sodium chondroitin sulfate, and methoxyethylene maleic anhydride copolymer.

7. The dentifrice composition according to any one of Claims 1 to 6, wherein the granules (D) are granules available by binding water insoluble powder materials with a water insoluble inorganic binder, granules available by coagulating only fine particles of calcium carbonate, granules available by binding water insoluble powder materials with a water insoluble organic binder or wet method silica granules having specific surface area, as measured by the BET method, of from 150 to 450 m$^2$/g.

**Patentansprüche**

1. Zahnputzmittelzusammensetzung, umfassend die folgenden Komponenten (A), (B) und (D):

(A) Pulvercellulose;
(B) ein Tensid, und
(D) ein Granulat mit einer Partikelgröße, die den Durchtritt durch ein 30-Mesh (JIS-Standard)-Sieb zulässt, aber die nicht den Durchtritt durch ein 200-Mesh-Sieb (JIS-Standard) zulässt,
wobei die Pulvercellulose (A) eine nicht-granulierte Pulvercellulose mit einer durchschnittlichen Partikelgröße von 10 bis 50 μm ist.

2. Zahnputzmittelzusammensetzung gemäß Anspruch 1, wobei der Gehalt an Pulvercellulose 0,2 bis 3 Gew-% beträgt.

3. Zahnputzmittelzusammensetzung gemäß Anspruch 1 oder Anspruch 2, wobei der Gehalt an Pulvercellulose 0,4 bis 2 Gew-% beträgt.

4. Zahnputzmittelzusammensetzung gemäß einem der Ansprüche 1 bis 3, wobei die Pulvercellulose einen durchschnittlichen Polymerisationsgrad von 350 bis 2250 aufweist.

5. Zahnputzmittelzusammensetzung gemäß einem der Ansprüche 1 bis 4, zusätzlich umfassend ein Bindemittel.

6. Zahnputzmittelzusammensetzung gemäß Anspruch 5, wobei das Bindemittel mindestens zwei Bindemittel umfasst, die aus der Gruppe ausgewählt sind, die aus Natriumalginat, Natriumcarboxymethylcellulose, Carrageenan, Xanthangummi, Natriumpolyacrylat, Hydroxyethylcellulose, Hydroxypropylcellulose, Pektin, Tragantgummi, Gummi arabicum, Guargummi, Karayagummi, Johannisbrotgummi, Gellangummi, Tamarindengummi, Psylliumsamengummi, Polyvinylalkohol, Natriumchondroitinsulfat und Methoxyethylen-Maleinsäureanhydrid-Copolymer besteht.

7. Zahnputzmittelzusammensetzung gemäß einem der Ansprüche 1 bis 6, wobei das Granulat (D) ein Granulat, erhältlich durch Binden von wasserunlöslichen Pulvermaterialien mit einem wasserunlöslichen anorganischen Bindemittel, ein Granulat, erhältlich durch Koagulieren von nur Feinpartikeln von Calciumcarbonat, ein Granulat, erhältlich durch Binden von wasserunlöslichen Pulvermaterialien mit einem wasserunlöslichen organischen Bindemittel, oder ein Nassverfahren-Silica-Granulat mit einer spezifischen Oberfläche, gemessen nach der BET Methode, von 150 bis 450 m$^2$/g ist.

**Revendications**

1. Composition de dentifrice comprenant les composants (A), (B) et (D) suivants :

(A) de la cellulose en poudre ;
(B) un tensio-actif, et
(D) des granulés ayant une taille de particule permettant le passage d'un tamis à maille de 30 (standard JIS) mais ne permettant pas le passage d'un tamis à maille de 200 (standard JIS),
dans laquelle la cellulose en poudre (A) est une poudre de cellulose non granulée ayant une taille de particule moyenne de 10 à 50 μm.

2. Composition de dentifrice selon la revendication 1, dans laquelle la teneur de la cellulose en poudre est de 0,2 à 3 % en poids.

**3.** Composition de dentifrice selon la revendication 1 ou la revendication 2, dans laquelle la teneur de la cellulose en poudre est de 0,4 à 2 % en poids.

**4.** Composition de dentifrice selon l'une quelconque des revendications 1 à 3, dans laquelle la cellulose en poudre a un degré de polymérisation moyen de 350 à 2250.

**5.** Composition de dentifrice selon l'une quelconque des revendications 1 à 4, comprenant en outre un liant.

**6.** Composition de dentifrice selon la revendication 5, dans laquelle le liant comprend au moins deux liants sélectionnés dans le groupe constitué de l'alginate de sodium, de la carboxyméthylcellulose de sodium, du carraghénane, de la gomme xanthane, du polyacrylate de sodium, de l'hydroxyéthylcellulose, de l'hydroxypropylcellulose, de la pectine, de la gomme adragante, de la gomme arabique, de la gomme de guar, de la gomme de karaya, de la gomme de caroube, de la gomme gellane, de la gomme de tamarin, de la gomme de graines de Psyllium, de l'alcool polyvinylique, du sulfate de chondroïtine de sodium et du copolymère d'anhydride maléique et méthoxyéthylène.

**7.** Composition de dentifrice selon l'une quelconque des revendications 1 à 6, dans laquelle les granulés (D) sont des granulés disponibles en liant des matériaux en poudre insolubles dans l'eau avec un liant inorganique insoluble dans l'eau, des granulés disponibles en coagulant seulement de fines particules de carbonate de calcium, des granulés disponibles en liant des matériaux en poudre insolubles dans l'eau avec un liant organique insoluble dans l'eau ou des granulés de silice de méthode humide ayant une aire de surface spécifique, telle que mesurée par la méthode BET, de 150 à 450 m$^2$/g.

[FIG. 1] 1/3

Oscillation

Expanded

Video tape magnetic layer

R4

Region to be evaluated
(= 2πR × 5 mm width)

Plaque model (video tape)

Interdental space model

[FIG. 2] 1/3

■ Not containing powder cellulose
☐ Containing powder cellulose

[FIG. 3]/3

**EP 2 548 547 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 55098111 A
- JP 9040537 A
- JP 11209255 A
- EP 0480172 A1
- WO 9534275 A1
- GB 2252042 A
- JP 1299211 A
- JP 9012436 A
- JP 6021053 B
- JP 3170250 B
- JP 4243815 A